# EUROPÄISCHE PATENTSCHRIFT

(11) **EP 2 421 648 B1**
(45) Veröffentlichungstag und Bekanntmachung des Hinweises auf die Patenterteilung: **05.03.2014**
(21) Anmeldenummer: 10713976.8
(22) Anmeldetag: 19.04.2010
(51) Int. Cl.: B01J 29/70, B01J 37/02, C07C 209/60

(54) **KATALYSATOREN UND VERFAHREN ZUR HYDROAMINIERUNG VON OLEFINEN**
CATALYSTS AND METHOD FOR THE HYDROAMINATION OF OLEFINS
CATALYSEURS ET PROCÉDÉ POUR L'HYDROAMINATION D'OLÉFINES

(30) Priorität: 22.04.2009 EP 09158472
(43) Veröffentlichungstag der Anmeldung: 29.02.2012
(73) Patentinhaber: BASF SE, 67056 Ludwigshafen (DE)
(72) Erfinder: HEIDEMANN, Thomas, 68519 Viernheim (DE); KEHRER, Jens, 67117 Limburgerhof (DE)
(86) Internationale Anmeldenummer: PCT/EP2010/055078
(87) Internationale Veröffentlichungsnummer: WO 2010/121974

(56) Entgegenhaltungen:
- EP-A1- 1 462 165
- WO-A1-97/07088
- WO-A1-2009/124924
- US-A- 5 114 565
- US-A- 5 744 667

## Beschreibung

Die vorliegende Erfindung betrifft Hydroaminierungskatalysatoren, deren Herstellung und Verwendung. Ein weiterer Gegenstand der vorliegenden Erfindung ist ein Verfahren zur Herstellung von Aminen durch Hydroaminierung von Olefinen sowie die Verwendung des mittels des efindungsgemäßen Verfahrens hergestellten tertiär-Butylamins.

In der WO 97/07088 ist ein Verfahren zur Herstellung on Olefinen an Bor-Beta Zeolithen offenbart. Es wird beschrieben, dass die Zeolithe modifiziert werden können, um beispielsweise die Selektivität, die Standzeit oder die Anzahl der möglichen Regenerierungen zu erhöhen. Eine mögliche Modifizierung der Hydroaminierungskatalysatoren besteht offenbarungsgemäß darin, dass man die Zeolithe mit Alkalimetallen, wie Na und K, Erdalkalimetallen, wie Ca und Mg, Erdmetallen, wie TI, Übergangsmetallen, wie z.B. Mn, Fe, Mo, Cu, Zn, Cr, Edelmetallen und/oder Seltenen Erdmetallen, wie z.B. La, Ce oder Y ionenaustauschen bzw. dotieren kann. Die Dotierung mit dem Element Li wird nicht explizit genannt.

Die US 5,114,565 offenbart das katalytische Reforming von Kohlenwasserstoffen mit großporigen Bor-haltigen Zeolithen.

In der US 5,744,667 werden Olefine, wie Trimethylpenten, zu den den entsprechenden Paraffinen, wie Trimethylpentan, umgesetzt. Der eingesetzte Katalysator enthält Platin, welches auf einem mit Alkalimetall-Kationen gesättigten Bor-Beta- Zeolithen als Trä aufgebracht wurde.

Die EP 1 462 165 offenbart einen einem thermisch aktivierten, calcinierten zeolithischen Katalysator,sowie ein Verfahren zur Herstellung von Alkylaminen durch Umsetzung von Olefinen mit Ammoniak, primären oder sekundären Aminen unter hydroaminierenden Bedingungen an einem solchen Katalysator.

Die WO 97/07088 offenbart ein Verfahren zur Herstellung von Aminen bedeuten, durch Umsetzung von Olefinen mit Ammoniak oder primären oder sekundären bei Temperaturen von 200 bis 350 °C und Drücken von 100 bis 300 bar in Gegenwart von Bor- Beta-Zeolithen.

In der WO 2009/124924 wird die Vorbehandlung von Hydroaminierungskatalysatoren, wobei man den Hydroaminierungskatalysator vor der Umsetzung von Olefinen mit Ammoniak, einem primären oder sekundären Amin mit einem Ammoniak-haltigen Gemisch in Kontakt bringt, wobei das Ammoniakhaltige Gemisch weniger als 40 Gew.-% Olefin enthält.

Die Aufgabe der vorliegenden Erfindung bestand darin, die Ausbeute von Aminen bei der Umsetzung von Ammoniak oder primären oder sekundären Aminen mit Olefinen gegenüber dem Stand der Technik verbessern.

Zur Lösung dieser Aufgabe wurde ein Hydroaminierungskatalysator enthaltend Bor-Beta Zeolithe gefunden, der mit Lithium dotiert ist und das molare Verhältnis von Bor zu Lithium Atomen im Hydroaminierungskatalysator 5:1 bis 50:1 beträgt.

Der erfindungsgemäße Hydroaminierungskatalysator enthält Bor-Beta Zeolithe.

Die Herstellung von Bor-Beta Zeolithen wird in der Anmeldung WO-A-98/07088 beschrieben, auf deren Inhalt Bezug genommen wird.

Bor-Beta Zeolithe können z. B. auch nach Gaodeng Xuexiao Huaxue Xuebao (1993), 14(2), 159 bis 163 oder Gaodeng Xuexiao Huaxue Xuebao (1989), 10(7), 677 bis 682 oder WO-A-92/20446 hergestellt werden.

Der Hydroaminierungskatalysator kann vollständig aus Bor-Beta Zeolithen bestehen. Üblicherweise enthalten die erfindungsgemäßen Hydroaminierungskatalysatoren noch Bindemittel, die zur Herstellung von Katalysatorformkörpern erforderlich sind.

Die Hydroaminierungskatalysatoren können neben Bindemitteln auch noch weitere Hilfsstoffe enthalten, wie Porenbildner und Anteigmittel.

Der Anteil an Bor-Beta Zeolithen im eingesetzten Hydroaminierungskatalysator beträgt vorzugweise 10 bis 100 Gew.-%, vorzugsweise 25 bis 99 Gew.-% und besonders bevorzugt 40 bis 98 Gew.% bezogen auf die Masse des getrockneten und calcinierten Hydroaminierungskatalysators.

Die Hydroaminierungskatalysatoren können in Form von Pulver oder bevorzugt in Form von Formkörpern wie Strängen, Tabletten oder Splitt eingesetzt werden.

Gängige Verfahren zur Herstellung von Formkörpern sind beispielsweise Extrusion, Tablettieren, d.h. mechanisches Verpressen oder Pelletieren, d.h. Kompaktieren durch kreisförmige und/oder rotierende Bewegungen und werden beispielsweise in Ertl, Knözinger, Weitkamp: "Handbook of heterogenoeous catalysis", VCH Weinheim, 1997, Seiten 98 ff oder der US 4,388,288) beschrieben.

Zur Herstellung der Formkörper (Verformung) können 2 bis 60 Gew.-% (bezogen auf die zu verformende Masse) Bindemittel zugesetzt werden. Als Bindemittel eignen sich verschiedene Aluminiumoxide, bevorzugt Boehmit, amorphe Aluminosilikate mit einem molaren SiO₂/Al₂O₃-Verhältnis von 25:75 bis 95:5, Siliciumdioxid, bevorzugt hochdisperses SiO₂ wie zum Beispiel Silikasole, Gemische aus hochdispersem SiO₂ und hochdispersem Al₂O₃, hochdisperses TiO₂ sowie Tone.

Die Hydroaminierungskatalysatoren werden bevorzugt als Stränge mit Durchmessern von zum Beispiel 1 bis 4 mm oder als Tabletten mit zum Beispiel 3 bis 5 mm Durchmesser für die Hydroaminierung der Olefine eingesetzt. Weiterhin können die Hydroaminierungskatalysatoren bevorzugt als Splitt eingesetzt werden, der durch Zerkleinerung von Katalysatorformkörpern erhalten wird.

Nach der Verformung werden die Extrudate oder Presslinge üblicherweise bei 80 bis 150°C für 2 bis 16 Stunden getrocknet und anschließend vorzugsweise calciniert.

Die Calcinierung wird in der Regel bei einer Temperatur von mehr als 400°C durchgeführt, damit das Bindermaterial aushärtet. Die Maximaltemperatur ist im Allgemeinen beschränkt durch die Stabilität des Bor-Beta Zeolithen, der bei Temperaturen von mehr als 550°C seine Kristallinität verliert. Die Calcinierung wird in der Regel großtechnisch im Drehrohr bei einer Temperatur im Bereich von 400 bis 560°C und einer Verweilzeit von 2 bis 4 Stunden durchgeführt. Im Labor wird üblicherweise in einem Ofen gearbeitet bei einer Temperatur von 480 bis 520°C und einem Zeitraum von 2 bis 32 Stunden.

Zur Erhöhung der Selektivität, der Standzeit und der Anzahl der möglichen Katalysatorregenerierungen kann man verschiedene Modifizierungen an den Hydroaminierungskatalysatoren vornehmen.

Eine Möglichkeit der Modifizierung des Hydroaminierungskatalysators besteht darin, dass man das Material - verformt oder unverformt - einer Behandlung mit Säuren, wie Salzsäure (HCl), Flusssäure (HF), Phosphorsäure (H₃PO₄), Schwefelsäure (H₂SO₄), Oxalsäure (HO₂C-CO₂H) oder deren Gemischen unterwirft.

Durch die Behandlung mit Säuren wird der Bor-Beta Zeolith im Allgemeinen in die H-Form überführt.

Eine besondere Ausführungsform besteht darin, dass man den Hydroaminierungskatalysator vor seiner Verformung mit Flusssäure (0,001 bis 2 molar, bevorzugt 0,05 bis 0,5 molar) 1 bis 3 Stunden unter Rückfluss behandelt. Nach Abfiltrieren und Auswaschen wird in der Regel bei 100 bis 160°C getrocknet und bei 400 bis 550°C calciniert.

Eine weitere besondere Ausführungsform liegt in einer HCl-Behandlung der Heterogenkatalysatoren nach ihrer Verformung mit Bindemittel. Hierbei wird der Hydroaminierungskatalysator in der Regel 1 bis 3 Stunden bei Temperaturen zwischen 60 und 80°C mit einer 3 bis 25%igen, insbesondere mit einer 12 bis 20%igen Salzsäure behandelt, anschließend ausgewaschen, bei 100 bis 160°C getrocknet und bei 400 bis 550°C calciniert.

Eine andere Möglichkeit der Modifizierung des Hydroaminierungskatalysators ist der Austausch mit Ammoniumsalzen, zum Beispiel mit NH₄Cl, oder mit Mono-, Di- oder Polyaminen, wobei der Bor-Beta Zeolith im Allgemeinen in die Ammonium-Form überführt wird. Hierbei wird der mit Bindemittel verformte Hydroaminierungskatalysator in der Regel bei 60 bis 80°C mit 10 bis 25%iger, bevorzugt ca. 20%iger NH₄Cl-Lösung 2 Stunden kontinuierlich in gewichtsmäßiger Hydroaminierungskatalysator/Ammoniumchlorid-Lösung von 1:15 ausgetauscht und danach bei 100 bis 120°C getrocknet.

Der erfindungsgemäße Hydroaminierungskatalysator ist mit Lithium dotiert.

Die Dotierung erfolgt bevorzugt dadurch, dass man den unverformten oder verformten Hydroaminierungskatalysator, der Bor-Beta Zeolithe enthält, mit einer Flüssigkeit in Kontakt bringt, die Li-lonen enthält.

Als Flüssigkeit wird in der Regel eine Flüssigkeit verwendet, die in der Lage ist eine Lithiumionenquelle zu solvatisieren.

Bevorzugte Flüssigkeiten sind Wasser und polare organische Lösungsmittel, wie Alkohole, beispielsweise Methanol, Ethanol bzw. Iso-Propanol, Ether, beispielsweise THF, DMF, DMSO oder NMP. In einer besonders bevorzugten Ausführungsform ist die Flüssigkeit Wasser.

Als Lithiumionenquelle wird bevorzugt ein lösliches Lithiumsalz oder eine Lithiumverbindung eingesetzt, die in der verwendeten Flüssigkeit Li-Ionen bildet. Besonders bevorzugt wird als Lithiumionenquelle ein in der Flüssigkeit lösliches Lithiumsalz eingesetzt.

Bevorzugte Lithiumsalze sind LiOH, Li-Nitrat, Li-Halogenide, wie LiCl, LiBr, LiF und Lil und Li-Carboxylate, wie Li-Oxalat, Li-Formiat, Li-Acetat, Li-Oxalat, Li-Citrat. Besonders bevorzugte Lithiumsalze sind LiOH, Li-Nitrat, LiCl, Li-Citrat und Li-Oxalat.

Lithiumverbindungen, die in einer Flüssigkeit Li-Ionen bilden können sind beispielsweise Organolithiumverbindungen, zum Beispiel Aryllithium-Verbindungen oder Alkyllithium-Verbindungen, wie Butyllithium, das beispielsweise in Wasser zu LiOH und Butan reagiert, wobei LiOH in Wasser Li-Ionen bildet.

Weitere Lithiumverbindungen, die in einer Flüssigkeit Li-Ionen bilden können sind Lithium-Alkoholate, wie Li-Methanolat, Li-Ethanolat oder Li-Propanolat.

Die Konzentration von Li-Ionen in der Flüssigkeit beträgt vorzugsweise 0,01 bis 100 Mol Li-Ionen pro Liter Flüssigkeit, bevorzugt 0,1 bis 10 Mol Li-Ionen pro Liter Flüssigkeit und besonders bevorzugt 0,2 bis5 Mol Li-Ionen pro Liter Flüssigkeit.

In einer bevorzugten Ausführungsform erfolgt die Dotierung der Bor-Beta Zeolithe durch Ionenaustausch.

In einer bevorzugten Ausführungsform erfolgt der Ionenaustausch dadurch, dass man die Bor-Beta Zeolithe in einem Strömungsrohr vorlegt und bei 20 bis 100°C eine Flüssigkeit, die Li-Ionen enthält, darüber leitet.

In einer weiteren bevorzugten Ausführungsform erfolgt die Dotierung durch Ionenaustausch dadurch, dass man den Bor-Beta Zeolithen in wässriger oder alkoholischer Lösung imprägniert bzw. tränkt. Die Tränkung des Bor-Beta Zeolithen kann nach den üblichen Verfahren erfolgen (A. B. Stiles, Catalyst Manufacture - Laboratory and Commercial Preperations, Marcel Dekker, New York, 1983), beispielsweise durch Aufbringung einer Lösung, die Li-Ionen enthält, in einer oder mehreren Tränkstufen. Die Tränkung kann auch dadurch erfolgen, dass das Trägermaterial entsprechend seiner Flüssigkeitsaufnahmekapazität maximal bis zur Sättigung mit der Tränklösung befeuchtet wird. Die Tränkung kann aber auch in überstehender Lösung erfolgen.

Bei mehrstufigen Tränkverfahren ist es zweckmäßig, zwischen einzelnen Tränkschritten zu trocknen und ggf. zu calcinieren.

Ein derartiger Ionenaustausch kann zum Beispiel an der unmodifizierten Form, der H-Form oder der Ammonium- Form der Hydroaminierungskatalysatoren vorgenommen werden.

Mit Lithium dotierte Bor-Beta Zeotlithe, die zum Beispiel durch Ionenaustausch als oder durch Imprägnierung erhalten wurden, werden in der Regel im Anschluss an die Dotierung wie voranstehend beschrieben getrocknet und/oder calciniert.

Das molare Verhältnis von Bor:Lithium-Atomen im erfindungsgemäßen Hydroaminierungskatalysator beträgt erfindungsgemäß 5:1 bis 50:1.

Das molare Verhältnis Bor und Lithium Atomen zueinander kann mittels bekannten Methoden der Elementaranalyse, beispielsweise der Atomabsorptionsspektrometrie (AAS), der Atomemissionsspektrometrie (AES), der Röntgenfluoreszenzanalyse (RFA) oder der ICP-OES (Inductively Coupled Plasma Optical Emission Spectrometry) gemessen werden.

Die erfindungsgemäßen Hydroaminierungskatalysatoren werden bevorzugt in einem Verfahren zur Herstellung von Aminen durch Umsetzung von Ammoniak oder primären oder sekundären Aminen mit Olefinen bei erhöhten Temperaturen und Drücken eingesetzt. Ganz besonders bevorzugt werden die erfindungsgemäßen Katalysatoren in einem Verfahren zur Herstellung von tert.-Butylamin eingesetzt.

Ein weiterer Gegenstand der vorliegenden Anmeldung ist deshalb ein Verfahren zur Herstellung von Aminen durch Umsetzung von Ammoniak oder primären oder sekundären Aminen mit Olefinen bei erhöhten Temperaturen und Drücken in Gegenwart von Bor-Beta Zeolithen, die mit Li dotiert sind.

Bor-Beta Zeolithe, die mit Li dotiert sind, können wir voranstehend beschrieben hergestellt werden.

Im erfindungsgemäßen Verfahren werden Ammoniak, primäre oder sekundäre Amine eingesetzt. Die primären oder sekundären Amine weisen dabei vorzugsweise C₁₋₂₀-Alkylreste, besonders bevorzugt C₁₋₆-Alkylreste, insbesondere Methylreste oder Ethylreste auf.

Neben Ammoniak sind ganz besonders bevorzugte Amine Monomethylamin, Dimethylamin, Monoethylamin, Diethylamin, n-Butylamin, Isopropylamin, Diisopropylamin und Di-n-butylamin. In einer besonders bevorzugten Ausführungsform wird als Ammoniak eingesetzt.

In das erfindungsgemäße Verfahren werden weiterhin Olefine eingesetzt.

Als Olefine können vorzugsweise C₂₋₂₀-Olefine eingesetzt werden, die aliphatisch sind. Sie können dabei linear oder verzweigt sein. Vorzugsweise werden C₂₋₁₂-Olefine, insbesondere C₂₋₆-Olefine eingesetzt. Beispiele geeigneter Olefine sind Ethen, Propen, Buten, Isobuten wie auch 1,3-Butadien. In einer besonders bevorzugten Ausführungsform wird als Olefin Isobuten eingesetzt.

Durch die Umsetzung von Ammoniak, primäre oder sekundäre Amine mit einem Olefin wird ein Hydroaminierungsprodukt erhalten.

Die mittels des erfindungsgemäßen Verfahrens bevorzugt herstellbaren Hydroaminierungsprodukte sind
ausgehend von Ethen und Ammoniak Mono-, Di- und/oder Triethylamin,
ausgehend von Ethen und Monoethylamin: Di- und/oder Triethylamin,
ausgehend von Isobuten und Ammoniak: tert.-Butylamin,
ausgehend von 1,3-Butadien und Ammoniak: 1-Amino-3-buten und/oder 2-Amino-3-buten,
ausgehend von 1,3-Butadien und n-Butylamin: (2-Butenyl)-n-butylamin und/oder (3-Butenyl)-n-butylamin und
ausgehend von Propen und Isopropylamin: Diisopropylamin.

In einer besonders bevorzugten Ausführungsform ist das Hydroaminierungsprodukt tert.-Butylamin, ausgehend von Isobuten und Ammoniak.

Die Umsetzung des Olefins mit Ammoniak und/oder dem primären oder sekundärem Amin in Gegenwart eines Bor-Beta Zeolithen kann zum Beispiel wie in EP-A 132 736, EP-A 752 409, EP-A 822 179 und WO-A-02/00597 beschrieben erfolgen.

Die Reaktion kann kontinuierlich, in Batch-Fahrweise oder in Semi-Batch-Fahrweise betrieben werden.

Bevorzugt geht man dabei in der Regel so vor, dass man Ammoniak und/oder primäres Amin oder gegebenenfalls sekundäres Amin zusammen mit Olefin in einem molaren Verhältnis von 1:1 bis 10:1, bevorzugt 1:1 bis 5:1, insbesondere bevorzugt 1:1 bis 3:1, mischt und in einem Festbett- oder in einer Wirbelschichtreaktor, der den erfindungsgemäßen Hydroaminierungskatalysator enthält, bei einem Druck von 40 bis 700 bar abs., bevorzugt 200 bis 300 bar abs., und einer Temperatur von 80 bis 400°C, bevorzugt 230 bis 320°C, in der Gasphase oder im überkritischen Zustand umsetzt.

Alternativ kann die Reaktion in der Flüssigphase bei einem Druck von 40 bis 80 bar abs. und einer Temperatur von 60 bis 120°C in einem Fest-Flüssig-Fließbett- oder einem Strömungsrohrreaktor, der den erfindungsgemäßen Hydroaminierungskatalysator enthält, durchgeführt werden.

Eine besondere Ausführungsform dieses Verfahrens besteht darin, dass man Ammoniak und/oder das primäre bzw. sekundäre Amin zusammen mit dem Olefin oder dem Olefingemisch im molaren Verhältnis 1:1 bis 5:1, bevorzugt 1:1 bis 3:1, gemischt einem Festbettreaktor, der den erfindungsgemäßen Hydroaminierungskatalysator enthält, zuführt und bei einem Druck von 100 bis 320 bar abs., bevorzugt 150 bis 310 bar abs., insbesondere 200 bis 300 bar abs., und einer Temperatur von 200 bis 350°C, bevorzugt 220 bis 330°C, insbesondere 230 bis 320°C, in der Gasphase oder im überkritischen Zustand umsetzt.

Die Lage des Gleichgewichts und damit der Umsatz zum gewünschten Hydroaminierungsprodukt ist stark vom gewählten Reaktionsdruck abhängig. Hoher Druck begünstigt das Additionsprodukt, doch stellt im Allgemeinen aus technischen und wirtschaftlichen Gründen der Druckbereich bis 300 bar abs. ein Optimum dar. Die Selektivität der Reaktion wird - neben Größen wie Ammoniak-/Amin-Überschuss und Katalysator - in hohem Maß durch die Temperatur beeinflusst. Zwar nimmt die Reaktionsgeschwindigkeit der Additionsreaktion mit steigender Temperatur stark zu, doch werden gegebenenfalls selektivitätsmindernde Nebenreaktionen gleichzeitig gefördert. Zudem ist eine Temperaturerhöhung aus thermodynamischer Sicht meist nicht vorteilhaft. Die Lage des Temperaturoptimums bezüglich Umsatz und Selektivität ist von der Konstitution des Olefins, des eingesetzten primären Amins und des Katalysators abhängig und liegt meist im Bereich von 220 bis 320°C.

Nach der Umsetzung wird das Produkt der Hydroaminierungsreaktion üblicherweise, z. B. durch Destillation, Rektifikation, Filtration, Wasserwäsche oder Adsorption, getrennt. Nicht umgesetzte Edukte oder zugeführte Inertgase können in die Reaktion zurückgeführt werden.

Das erfindungsgemäß hergestellte tert.-Butylamin kann als Rohstoff in der Gummiindustrie (Vulkanisationsbeschleuniger) oder zur Herstellung von Pflanzenschutzmitteln oder Pharmazeutika verwendet werden.

Die erfindungsgemäßen Hydroaminierungskatalysatoren ermöglichen eine Verbesserung der Ausbeute von Aminen bei der Umsetzung von Ammoniak oder primären oder sekundären Aminen mit Olefinen gegenüber dem Stand der Technik. Die erfindungsgemäßen Katalysatoren sind im großtechnischen Maßstab herstellbar und weisen eine hohe Standzeit auf. Mit dem erfindungsgemäßen Katalysator ist im Allgemeinen bei gleichem Umsatz eine höhere Katalysatorbelastung oder bei gleicher Katalysatorbelastung ein höherer Umsatz möglich.

Die Erfindung wird durch die nachstehenden Beispiele näher erläutert.

### Beispiele:

### Katalysatorsynthese

### Beispiel 1: Herstellung eines Bor-Beta-Zeolithformkörper

133 g Bor-Beta-Zeolith (Verhältnis von SiO₂:B₂O₃ = 20, Synthese gemäß WO 97/07088) wurden mit 67 g Boehmit und 4 g Ameisensäure versetzt. Im Kneter wurde die Mischung kompaktiert und unter vorsichtigem Wasserzusatz (110 ml) verknetet. Die Knetzeit betrug 60 min. In einer Strangpresse wurden mit einem Pressdruck von 100 bar 2,5 mm-Stränge erzeugt, bei 110°C 16 h getrocknet und anschließend 16 h bei 500°C kalziniert. Der Borgehalt dieser so hergestellten Stränge betrug 100 mmol pro 100 g Stränge, die Wasseraufnahme 0,6 ml/g.

### Beispiel 2: Dotierung des Bor-Beta-Zeolithformkörpers mit Li

100 g Stränge wurden im Rotationsverdampfer bei 20°C unter Rotieren (20 Umdrehungen/min) mit einer Lösung aus 0,73 g LiNO₃ (95%ig = 10 mmol Li) in 60 ml Wasser getränkt. Nach kurzem Antrocknen bei 50°C i.Vak. wurden die getränkten Stränge 12 h bei 120°C im Trockenschrank getrocknet und anschließend 8 h bei 450°C im Drehrohrofen unter Durchleiten von 50 Nl/h Luft calciniert. Anschließend wurden die Stränge zerkleinert und eine Splittfraktion mit 1-1,6 mm Durchmesser abgesiebt.

### Beispiel 3: Dotierung des Bor-Beta-Zeolithformkörpers mit Li

Man verfuhr wie unter Beispiel 1 mit der Variation, dass 0,36 g LiNO₃ (5 mmol Li) eingesetzt wurden.

### Beispiel 4: Dotierung des Bor-Beta-Zeolithformkörpers mit Li

Man verfuhr wie unter Beispiel 1 mit der Variation, dass 1,10 g LiNO₃ (15 mmol Li) eingesetzt wurden.

### Beispiel 5: Dotierung des Bor-Beta-Zeolithformkörpers mit Li

Man verfuhr wie unter Beispiel 1 mit der Variation, dass 1,46 g LiNO₃ (20 mmol Li) eingesetzt wurden.

### Beispiel 6: Dotierung des Bor-Beta-Zeolithformkörpers mit Li

Man verfuhr wie unter Beispiel 1 mit der Variation, dass 3,6 g LiNO₃ (50 mmol Li) eingesetzt wurden.

### Beispiel 7: Dotierung des Bor-Beta-Zeolithformkörpers mit Na

Man verfuhr wie unter Beispiel 1 mit der Variation, dass 0,85 g NaNO₃ (10 mmol Na) eingesetzt wurden.

### Beispiel 8: Dotierung des Bor-Beta-Zeolithformkörpers mit Na

Man verfuhr wie unter Beispiel 1 mit der Variation, dass 1,70 g NaNO₃ (20 mmol Na) eingesetzt wurden.

### Beispiel 9: Dotierung des Bor-Beta-Zeolithformkörpers mit Rb

Man verfuhr wie unter Beispiel 1 mit der Variation, dass 1,48 g RbNO₃ (10 mmol Rb) eingesetzt wurden.

### Beispiel 10: Dotierung des Bor-Beta-Zeolithformkörpers mit Cs

Man verfuhr wie unter Beispiel 1 mit der Variation, dass 1,95 g CsNO₃ (10 mmol Cs) eingesetzt wurden.

### Beispiel 11: Dotierung des Bor-Beta-Zeolithformkörpers mit K

Man verfuhr wie unter Beispiel 1 mit der Variation, dass 0,51g KNO₃ (5 mmol K) eingesetzt wurden.

### Beispiel 12: Dotierung des Bor-Beta-Zeolithformkörpers mit K

Man verfuhr wie unter Beispiel 1 mit der Variation, dass 1,01 g KNO₃ (10 mmol K) eingesetzt wurden.

### Beispiel 13: Dotierung des Bor-Beta-Zeolithformkörpers mit K

Man verfuhr wie unter Beispiel 1 mit der Variation, dass 2,02 g KNO₃ (20 mmol) eingesetzt wurden.

### Herstellung von tert.-Butylamin

In einem Rohrreaktor (6 mm Innendurchmesser) wurden 10 g Katalysatorsplitt unter isothermen Bedingungen bei 270°C und einem Druck von 270 bar mit 43 g/h eines Gemisches aus Isobuten und NH₃ (1 mol:1,5 mol) beaufschlagt und die Umsetzung mittels on-line GC verfolgt.

In nachfolgender Tabelle sind die Ergebnisse für oben beschriebene Katalysatoren (Beispiele 1 bis 13) nach jeweils 48 h Laufzeit aufgelistet:

| Beispiel | | Sel. tBA | Ausbeute tBA | molares Verhaltnis von B:Alkali im Hydroaminierungskatalysator |
|---|---|---|---|---|
| | | | q tBA/g feed | |
| 1 | B-Beta | >99% | 14,0 | - |
| 2 | 10mmol Li | >99% | 15,0 | 10:1 |
| 3 | 5mmol Li | >99% | 15,1 | 20:1 |
| 4 | 15mmol Li | >99% | 15,0 | 20:3 |
| 5 | 20mmol Li | >99% | 14,9 | 5:1 |
| 6 | 50mmol Li | >99% | 13,2 | 2:1 |
| 7 | 10mmol Na | >99% | 14,5 | 10:1 |
| 8 | 20mmol Na | >99% | 14,3 | 5:1 |
| 9 | 10mmol Rb | >99% | 14,3 | 10:1 |
| 10 | 10mmol Cs | >99% | 14,1 | 10:1 |
| 11 | 5mmol K | >99% | 13,9 | 20:1 |
| 12 | 10mmol K | >99% | 14,5 | 10:1 |
| 13 | 20mmol K | >99% | 13,7 | 5:1 |

Man erkennt, dass eine Alkalidotierung mit Na, K, Rb, Cs gegenüber dem Einsatz von undotiertem B-Beta zu keiner oder nur einer geringen Ausbeutesteigerung von max. 0,5% führt, während durch Dotierung mit Li eine Steigerung der Ausbeute um 0,9-1,1% zu erreichen ist.

## Patentansprüche

1. Hydroaminierungskatalysator enthaltend Bor-Beta Zeolithe, **dadurch gekennzeichnet, dass** der Hydroaminierungskatalysator mit Lithium dotiert ist und das molare Verhältnis von Bor zu Lithium-Atomen im Hydroaminierungskatalysator 5:1 bis 50:1 beträgt.

2. Hydroaminierungskatalysator gemäß Anspruch 1, **dadurch gekennzeichnet, dass** die Dotierung dadurch erfolgt, dass man den Hydroaminierungskatalysator mit einer Flüssigkeit in Kontakt bringt, die Li-Ionen enthält.

3. Hydroaminierungskatalysator gemäß Anspruch 2, **dadurch gekennzeichnet, dass** die Konzentration von Li-Ionen in der Flüssigkeit 0,01 bis 100 Mol Li-Ionen pro Liter Flüssigkeit beträgt.

4. Hydroaminierungskatalysator gemäß mindestens einem der Ansprüche 1 bis 3, **dadurch gekennzeichnet, dass** die Dotierung durch Ionenaustausch oder Tränkung erfolgt.

5. Hydroaminierungskatalysator gemäß mindestens einem der Ansprüche 1 bis 4, **dadurch gekennzeichnet, dass** der Anteil von Bor-Beta Zeolithen im Hydroaminierungskatalysator 10 bis 100 Gew.% beträgt.

6. Verfahren zur Herstellung von Aminen durch Umsetzung von Ammoniak oder primären oder sekundären Aminen mit Olefinen bei erhöhten Temperaturen und Drücken in Gegenwart eines Hydroaminierungskatalysators gemäß einem der Ansprüche 1 bis 5.

7. Verfahren nach Anspruch 6, **dadurch gekennzeichnet, dass** das molare Verhältnis von Ammoniak, primären oder sekundären Aminen zu Olefin zwischen 1:1 und 3:1 beträgt.

8. Verfahren nach mindestens einem der Ansprüche 6 bis 7, **dadurch gekennzeichnet, dass** Olefine mit Ammoniak umgesetzt werden.

9. Verfahren nach mindestens einem der Ansprüche 6 bis 8, **dadurch gekennzeichnet, dass** man als Olefin Isobuten einsetzt.

10. Verfahren zur Herstellung von Rohstoffen für die Gummiindustrie, z.B. als Vulkanisationsbeschleuniger, oder zur Herstellung von Pflanzenschutzmitteln oder Pharmazeutika, **dadurch gekennzeichnet, dass** man zunächst tertiär-Butylamin in einem Verfahren gemäß den Ansprüchen 6 bis 9 herstellt und das so hergestellte tertiär-Butylamin in ein Verfahren zur Herstellung von Rohstoffen für die Gummiindustrie, z.B. als Vulkanisationsbeschleuniger, oder zur Herstellung von Pflanzenschutzmitteln oder Pharmazeutika einsetzt.

## Claims

1. A hydroamination catalyst comprising boron beta zeolites wherein the hydroamination catalyst is doped with lithium and the molar ratio of boron to lithium atoms in the hydroamination catalyst is from 5:1 to 50:1.

2. The hydroamination catalyst according to claim 1, wherein doping is effected by bringing the hydroamination catalyst into contact with a liquid comprising Li ions.

3. The hydroamination catalyst according to claim 2, wherein the concentration of Li ions in the liquid is from 0.01 to 100 mol of Li ions per liter of liquid.

4. The hydroamination catalyst according to at least one of claims 1 to 3, wherein doping is effected by ion exchange or impregnation.

5. The hydroamination catalyst according to at least one of claims 1 to 4, wherein the proportion of boron beta zeolites in the hydroamination catalyst is from 10 to 100% by weight.

6. A process for preparing amines by reaction of ammonia or primary or secondary amines with olefins at elevated temperatures and pressures in the presence of a hydroamination catalyst according to any of claims 1 to 5.

7. The process according to claim 6, wherein the molar ratio of ammonia, primary or secondary amines to olefin is in the range from 1:1 to 3:1.

8. The process according to at least one of claims 6 and 7, wherein olefins are reacted with ammonia.

9. The process according to at least one of claims 6 to 8, wherein isobutene is used as olefin.

10. A process for producing raw materials for the rubber industry, e.g. as vulcanization accelerator, or for producing crop protection agents or pharmaceuticals, which comprises firstly preparing tert-butylamine in a process according to claims 6 to 9 and using the tert-butylamine prepared in this way in a process for producing raw materials for the rubber industry, e.g. as vulcanization accelerator, or for producing crop protection agents or pharmaceuticals.

## Revendications

1. Catalyseur d'hydroamination contenant une zéolithe de bore de type bêta, **caractérisé en ce que** le catalyseur d'hydroamination est dopé au lithium et le rapport molaire de bore aux atomes de lithium dans le catalyseur d'hydroamination vaut 5:1 à 50:1.

2. Catalyseur d'hydroamination selon la revendication 1, **caractérisé en ce que** le dopage a lieu en ce qu'on met en contact le catalyseur d'hydroamination avec un liquide qui contient des ions de Li.

3. Catalyseur d'hydroamination selon la revendication 2, **caractérisé en ce que** la concentration en ions de Li dans le liquide vaut 0,01 à 100 moles d'ions de Li par litre de liquide.

4. Catalyseur d'hydroamination selon au moins l'une quelconque des revendications 1 à 3, **caractérisé en ce que** le dopage a lieu par échange ionique ou imprégnation.

5. Catalyseur d'hydroamination selon au moins l'une quelconque des revendications 1 à 4, **caractérisé en ce que** la proportion de zéolithes de bore de type bêta dans le catalyseur d'hydroamination vaut 10 à 100% en poids.

6. Procédé pour la préparation d'amines par transformation d'ammoniac ou d'amines primaires ou secondaires avec des oléfines à des températures et des pressions augmentées en présence d'un catalyseur d'hydroamination selon l'une quelconque des revendications 1 à 5.

7. Procédé selon la revendication 6, **caractérisé en ce que** le rapport molaire de l'ammoniac, des amines primaires ou des amines secondaires à l'oléfine vaut entre 1:1 et 3:1.

8. Procédé selon au moins l'une quelconque des revendications 6 à 7, **caractérisé en ce que** les oléfines sont transformées avec de l'ammoniac.

9. Procédé selon au moins l'une quelconque des revendications 6 à 8, **caractérisé en ce qu'**on utilise, comme oléfine, de l'isobutène.

10. Procédé pour la préparation de matières premières pour l'industrie du caoutchouc, par exemple comme accélérateur de vulcanisation, ou pour la préparation d'agents de phytoprotection ou de produits pharmaceutiques, **caractérisé en ce qu'**on prépare d'abord de la tert-butylamine dans un procédé selon les revendications 6 à 9 et on utilise la tert-butylamine ainsi préparée dans un procédé pour la préparation de matières premières pour l'industrie du caoutchouc, par exemple comme accélérateur de vulcanisation, ou pour la préparation d'agents de phytoprotection ou de produits pharmaceutiques.
